# EUROPEAN PATENT SPECIFICATION

(11) **EP 2 600 909 B1**
(45) Date of publication and mention of the grant of the patent: **19.11.2014**
(21) Application number: 11736453.9
(22) Date of filing: 04.07.2011
(51) Int. Cl.: A61L 15/26, A61L 15/46

(54) **A DRESSING DEVICE FOR USE WITH A CANNULA OR A CATHETER**
WUNDAUFLAGEVORRICHTUNG ZUR VERWENDUNG MIT EINER KANÜLE ODER EINEM KATHETER
DISPOSITIF DE TRAITEMENT DES PLAIES UTILISABLE AVEC UNE CANULE OU UN CATHÉTER

(30) Priority: 14.07.2010 US 344403 P
(43) Date of publication of application: 12.06.2013
(73) Proprietor: Bard Access Systems, Inc., Salt Lake City, UT 84116 (US)
(72) Inventor: REAL, Keith, Delgany, County Wicklow (IE); DONNELLAN, Peter, Kilmallock, County Limerick (IE); NÍ BEILLIÚ, Máire, Dundalk, County Louth. (IE)
(74) Representative: Hoffmann Eitle
(86) International application number: PCT/IE2011/000034
(87) International publication number: WO 2012/007929

(56) References cited:
- WO-A2-2007/127236
- WO-A2-2008/010199
- US-B1- 6 596 293

## Description

### Introduction

The present invention relates to a wound device, particularly for use with IV catheters and other percutaneous devices.

Vascular and nonvascular percutaneous medical devices such as: IV catheters, central venous lines, arterial catheters, dialysis catheters, peripherally inserted coronary catheters, mid-line catheters, drains, chest tubes, externally placed orthopedic pins, and epidural catheter tubes are widely used in modern day medical practice. Annually more than 20 million inpatients in hospitals in the United States receive intravenous therapy and almost 5 million require central venous catheterization (Bouza et al., 2002)

Mechanical complications such as haemorrhage and thrombosis are associated with catheterization. The risk of bleeding associated with catheterization is reported to range between 1% and 8% (Mital *et al.,* 2004) and although minor bleeding may be quite common serious bleeding is rare (Doerfler *et al.* 1996). Though dressings for antimicrobial effectiveness have long been available no product deals suffciently with the bleeding from these wound types and this leads to dressing changes being a regular occurrence. There remains a need for an effective dressing for use with IV catheters that stops bleeding and is an effective antimicrobial solution.

Catheter use causes a semi-permanent breach of the skin that provides an access point for pathogens to enter the body, placing the patient at risk for local and systemic infectious complications. The potential for infection may be increased by proliferation of bacteria within or underneath the dressing. Studies have shown that between 5% and 25% of IV devices are colonized at the time of removal (Maki et al., 1998). Skin flora is the main source of microbial contamination and is responsible for approximately 65% of catheter related infections. Bacteria from the skin migrate along the external surface of the catheter and colonize the intravascular catheter tip leading to catheter related blood stream infections (Raad *et* al., 2001; Sheretz *et al.,* 1997). Catheter-related bloodstream infection (CR-BSI) is the third most common health care-acquired infection in the United States and is considered one of the most dangerous complications for patients. In Europe the incidence and density of central venous catheter (CVC) related bloodstream infections ranges from 1 - 3.1 per 1000 patient days (Suetens *et al.,* 2007). Most organisms responsible for CR-BSIs originate from the insertion site of the catheter (Timsit, 2007), therefore, decreasing bacterial colonization at the site of insertion may help reduce the incidence of CR-BSIs.

In WO 2008/010199 A2, a mesh comprising nanofibers of oxidised polysaccharide and a fibre-forming polymer is disclosed. In this application, the oxidised polysaccharide is uniformly dispersed in the form of molecules and/or nanoparticles in a matrix of the fibre-forming polymer. The oxidised polysaccharide may be polyanhydroglucuronic acid or salts or intermolecular complexes thereof. The polymer may be PVA. The mesh may be used in wound dressings. The dressing may have multiple layers.

In US 6596293 B1, a method is disclosed for preparing a drug delivery material and device comprising cross-linking a biological polymer with a cross-linking agent and loading the cross-linked biopolymer with a bioactive agent. Preferred embodiments are disclosed wherein the drug delivery material is used in a catheter securing, drug delivery device, in a wound dressing, and in a wound dressing for percutaneous catheters.

WO 2007/127236 A2 discloses antimicrobial articles for use with a percutaneous device, comprising a matrix which may contact the percutaneous device in a three-dimensional mode and release antimicrobial agents (e.g., silver ions) to the percutaneous device access site. In addition, the application states that the antimicrobial article of the present invention may donate moisture to a dry dermal site (e.g., a dry wound bed) and/or absorb liquid or exudates of a dermal site. The application also discusses methods for treating and/or preventing an infection using said antimicrobial articles.

It is an object of the current invention to provide an improved wound dressing device that will provide protection at an insertion site.

### Summary of the Invention

According to the invention there is provided a dressing device for use with a transcutaneous medical device such as a cannula or a catheter, the dressing device comprising a flexible hydrophillic polyurethane matrix, an antimicrobial agent contained within the matrix, and a haemostatic agent contained within the matrix, the haemostatic agent comprising polyanhydroglucuconic acid or salt thereof in an amount to achieve a haemostatic effect, and the antimicrobial agent comprising chlorhexidine di-gluconate in an, amount to achieve an antimicrobial effect without adversely affecting wound healing, wherein the chlorhexidine di-gluconate is present in an amount of from 9% to 16% (w/w).

The invention provides a wound dressing device that prevents microbial colonization of the dressing and stops bleeding from the insertion site. The device provides combined haemostatic and antimicrobial effects at the insertion site but without adversely affecting wound healing.

This is a particularly surprising aspect of the invention because a dressing composition that contains only polyanhydroglucuronic acid or salt thereof to promote wound healing and haemostasis is not conducive to contamination and infection control. The addition of chlorhexidine di-gluconate as an antimicrobial agent effective at preventing contamination and infection would be expected to adversely affect wound healing. We have surprisingly found that this is not the case.

The polyanhydroglucuronic salt may be present in an amount of from 3% to 20% (w/w). The polyanhydroglucuronic salt may for example be present in an amount of approximately 8% w/w.

The chlorhexidine di-gluconate may for example be present in an amount of approximately 11% w/w.

In one embodiment the dressing device comprises approximately 8% (w/w) polyanhydroglucoronic acid, approximately 11% (w/w) chlorhexidine di-gluconate, and approximately 81% hydrophillic flexible polyurethane foam.

In one embodiment the dressing device comprises an aperture for reception of a medical device such as a cannula or a catheter.

In one embodiment the dressing device comprises a breathable backing material to allow vapour transmission from the device.

A skin contacting side of the device may contain an adhesive compound to keep the device affixed to a site.

In one case the central access aperture is a circular hole ranging in size from 0.1mm to 10mm in diameter.

Alternatively the central access aperture is "x" shaped.

The central access aperture may be "T" shaped.

In one embodiment the device contains a quantity of the antimicrobial agent to maintain antimicrobial efficiency for up to 7 days.

The invention in one aspect is an absorbent polymeric wound dressing containing a broad spectrum antimicrobial agent and a haemostatic agent with a moisture vapour permeable backing and radial slit and central access hole to allow insertion of an IV catheter line or other similar percutaneous device. The device contains sufficient quantities of the broad spectrum antimicrobial agent to ensure that a clear antimicrobial, zone of inhibition can be maintained around the insertion site and to prevent microbial contamination of the dressing. The device also contains sufficient quantities of haemostatic agent in order to successfully control minor bleeding at the insertion site.

The absorbent polymer foam matrix dressing of the invention rapidly addresses bleeding, prevents dermal wound site contamination and infection while at the same time promoting wound healing. Rapid wound healing and closure in a controlled aseptic (near microbe free) environment provides the optimal conditions for reduced wound site morbidity.

The absorbent polymer foam matrix dressing composition of the invention addresses the paradoxical requirement of good antimicrobial efficacy, good haemostatic efficacy and good wound healing properties in the same absorbent, conformable polymer foam composition containing specific narrow range non-antagonistic concentrations of antimicrobial, haemostatic and wound healing agents that allow for combined effective interactions that are antimicrobial, haemostatic and wound healing.

A specific application of the present invention relates to a wound device, particularly for use with IV catheters and other percutaneous devices.

The invention disclosure described herein identifies a novel device composition which allows for the singular important advantage in being able to attain antimicrobial, haemostatic and wound-healing promoting characteristics in a single absorbent and compliant device system. Normally achieving such functional heterogeneity in one device is not possible due to antagonistic effects of the separate functions on one another. The unique feature of this invention is that it is able to identify and integrate effective ranges for each active component without adversely affecting the functions of the other components.

### Brief Description of the Drawings

The invention will be more clearly understood from the following description of an embodiment thereof, given by way of example only, with reference to the accompanying drawings, in which:

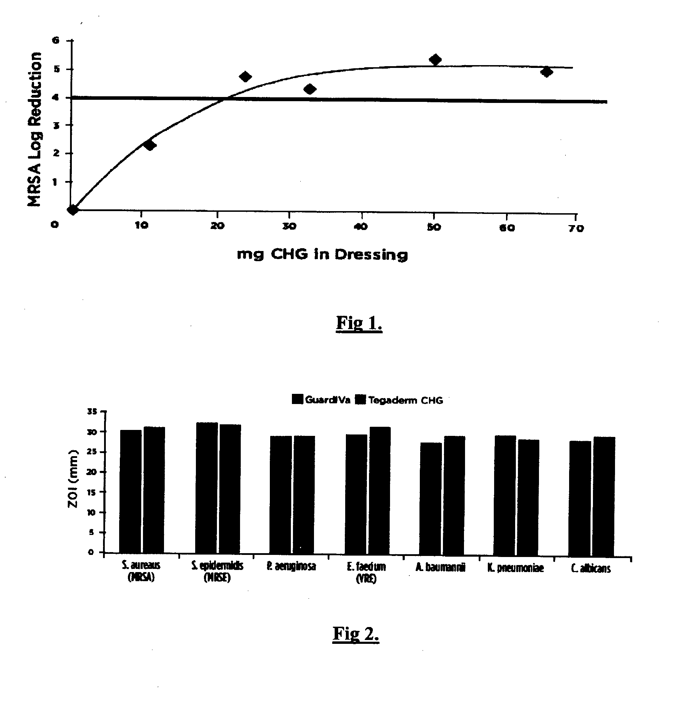

Fig. 1 shows the results of the antimicrobial testing against methicillin-resistant Staphylococcus aureus (MRSA) using AATCC Test Method 100-2004 after 24 hrs incubation of sterilized polyurethane foam matrix dressings containing polyanhydroglucuronic acid calcium sodium salt (8% w/w) dressing with increasing weight percent of chlorhexidine di-gluconate (CHG) in a 217 mg, 25 mm diameter dressing;
Fig. 2 shows results of Kirby Bauer antimicrobial zone of inhibition testing after 24 hours incubation for the polyurethane foam matrix described in example 3 and a commercially available chlorhexidine di-gluconate containing matrix control material (intended to reduce catheter related blood stream infection) against methicillin resistant Staphylococcus aureus (MRSA), methicillin resistant Staphylococcus epidermidis (MRSE), Pseudomonas aeruginosa, vancomycin resistant Enterococcus faecium (VRE), Acinetobacter baumannii, Klebsiella pneumoniae and Caridida albicans;

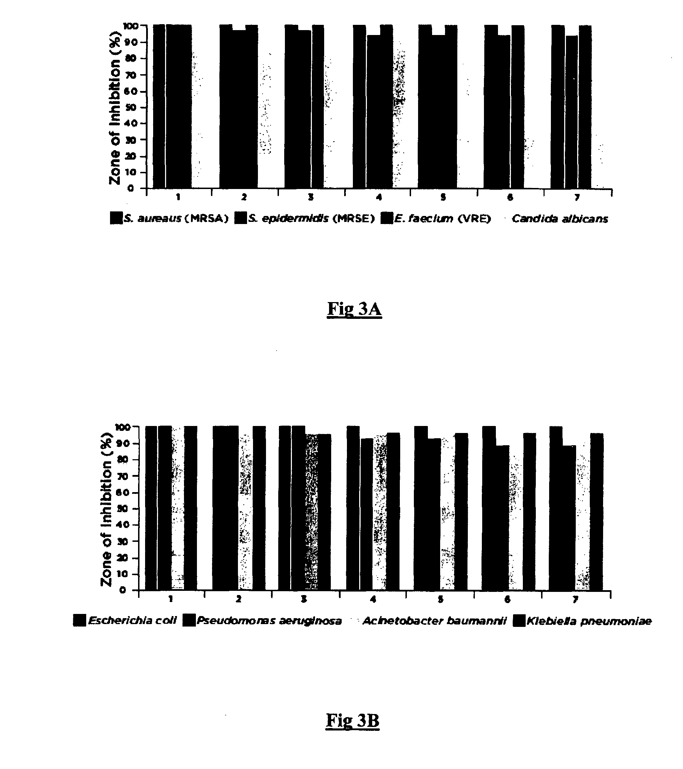

Fig. 3 shows results of Kirby Bauer antimicrobial zone of inhibition testing after 1, 2, 3, 4, 5, 6 & 7 days for the polyurethane foam matrix described in example 3 against the gram positive organisms (Fig 3A) methicillin resistant Staphylococcus aureus (MRSA), methicillin resistant Staphylococcus epidermidis (MRSE), vancomycin resistant Enterococcus faecium (VRE) and the fungus and Candida albicans, and against gram negative organisms (Fig 3B) Escherichia coli, Pseudomonas aeruginosa, Acinetobacter baumannii and Klebsiella pneumoniae;

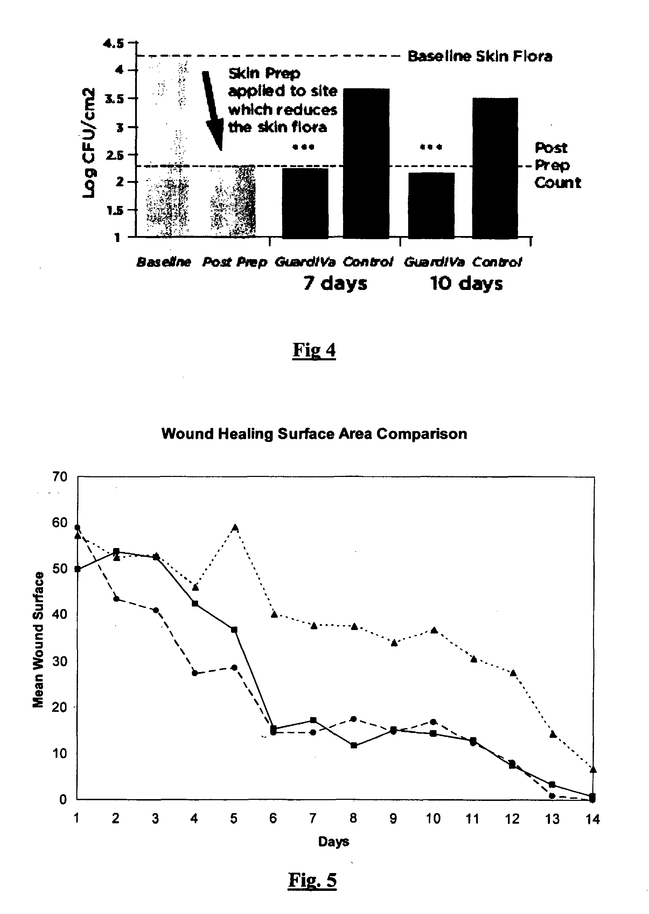

Fig. 4 shows the results of testing of suppression of re-growth of human skin microflora on prepped subclavian sites for the polyurethane foam matrix described in example 3 in healthy volunteers (N=12). The method of testing was based on that described by Maki et al 2008. The control dressing is polyurethane foam matrix with no chlorhexidine di-gluconate arid no polyanhydroglucutonic acid. Bacterial counts are expressed as log10 CFU/cm². There is a statistically significant difference (P < 0.001) between test and control dressings at both day 7 and day 10. Skin prepping was carried out for 1 minute with 70% isopropyl alcohol;
Fig 5 compares mean wound surface area in a study 1 of an untreated control (dashed line ( - - -) un-treated wounds), a test item (solid line - wounds treated with the test dressing device of Example 3), and a control dressing (dotted line (.....));

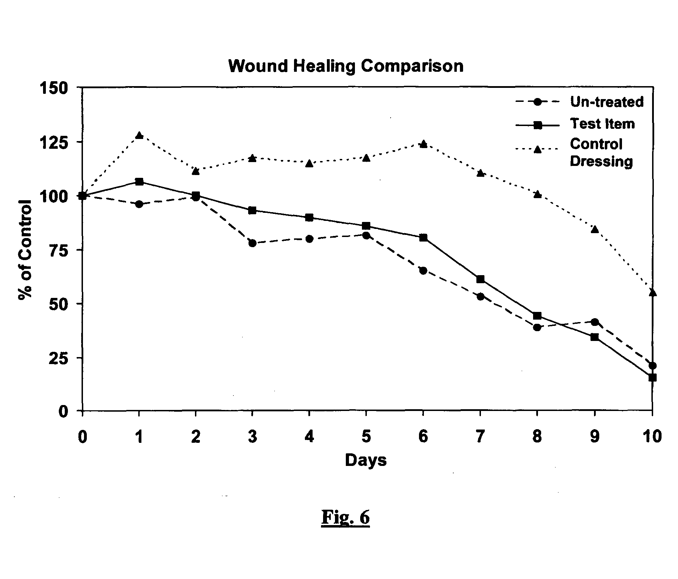

Fig. 6 demonstrates the change in wound surface area of an untreated control (dashed line (- - -) un-treated wounds), a test item (solid line - wounds treated with the test dressing device of Example 3), and a control dressing (dotted line (.....));

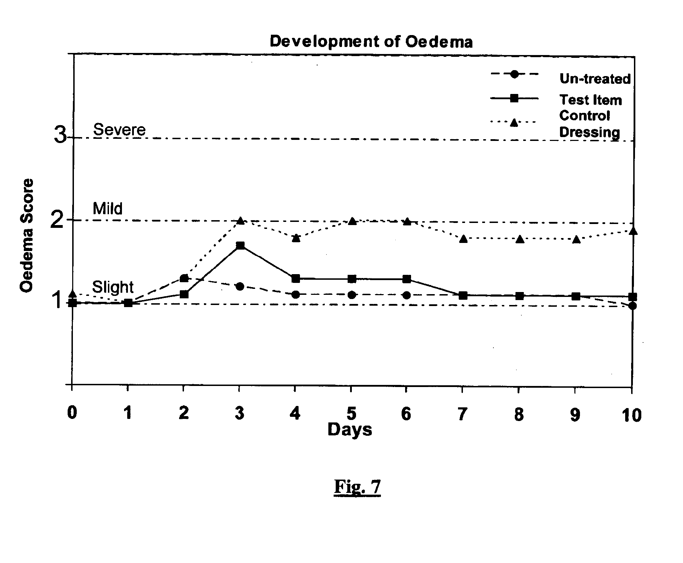

Fig. 7 demonstrates oedema development of an untreated control (dashed line (- - -) untreated wounds), a test item (solid line - wounds treated with the test dressing device of Example 3), and a control dressing (dotted line (.....));

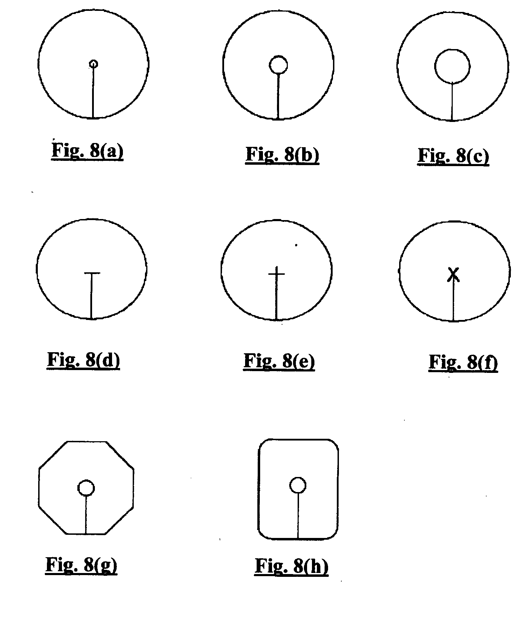

Fig. 8 illustrates of some of different physical embodiments of a wound dressing device of the invention; and

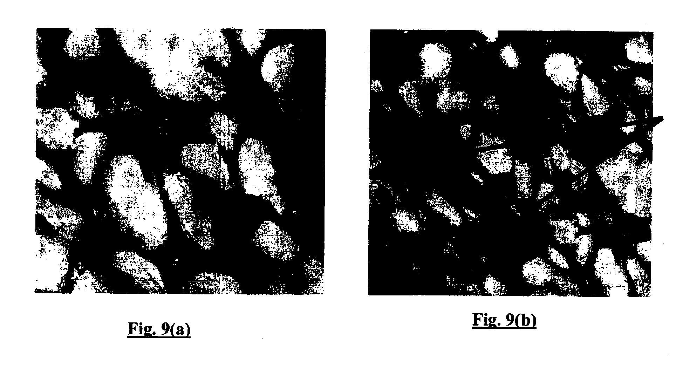

Fig. 9 shows representative micrographs of polyurethane foam A) without the impregnation of haemostatic calcium sodium polyanhydroglucuronic acid and B) with the impregnation of calcium sodium polyanhydroglucuronic acid. The apparatus in Fig. 9(b) indicate particles of calcium - sodium polyanhydroglucuronic acid.

### Detailed Description of the invention

The invention provides wound dressings for controlling minor bleeding at the access sites of IV catheters and similar percutaneous devices. Moreover the invention provides protection at the access site and contains a broad spectrum antimicrobial agent to help resist microbial colonization of the dressing. The device also successfully reduces bleeding time. The dressing device provides advantages over other IV site dressings as it contains a haemostatic agent.

In one embodiment of the invention polyanhydroglucuronic acid is incorporated into the polymeric base material as the haemostatic agent and chlorhexidine di-gluconate is incorporated as the broad spectrum antimicrobial agent, wherein the chlorhexidine di-gluconate is present in an amount of from 9% to 16% (w/w).

The device may have a moisture vapour permeable backing to allow for moisture transmission. The backing may, for example, comprise a thin polyurethane film.

The system of the present invention has been shown to effectively maintain antimicrobial, efficacy over a period of up to 7 days.

On complete saturation with an aqueous medium the absorption capacity of the foam of the present invention is typically greater than 8 times (wt / wt relative to the dry weight of the dressing). Preferred absorption capacity of the dressing is 10 to 15 times (wt / wt).

In one embodiment of the system the polymeric base material is polyurethane foam. The components that make up the system may be present in the system with final concentrations of, for example, 8% (w/w) polyanhydroglucoronic acid 11 % (w/w) chlorhexidine di-gluconate and 81% hydrophillic flexible polyurethane foam.

The dressings of the invention will generally be sterile. Sterilization may be carried out using gamma irradiation but other sterilization methods such as ethylene oxide sterilization may also be used.

In one embodiment, the dressing device has an adhesive technology on the skin contacting surface to aid in site securement and also for removal and re-securement.

In one embodiment the wound dressing is circular with an outer diameter of 0.6 to 2 inches (1.52 to 5.08cm). The outer diameter may be about one inch (2.54cm). The dressing of the invention will typically have a central access aperture to facilitate passage of an IV catheter line or other similar percutaneous device.

In other embodiments the central access aperture may be "x" or "T" shaped. One embodiment of the device has a circular cut central access site. The size of the central access site may vary from typically 1 mm to 10 mm.

In further embodiments of the invention the device may be non circular.

Having described the invention in general terms, reference is now made to specific non-limiting examples.

The invention provides a haemostatic and wound-healing promoting antimicrobial dressing for general wound use, but also particularly for controlling minor bleeding at the access sites of IV catheters and similar percutaneous devices. Moreover the invention promotes wound healing while providing protection at the access site by slow release of a broad-spectrum antimicrobial agent to help resist microbial colonization of the dressing. The device also successfully reduces bleeding time. The antimicrobial, haemostatic dressing device described herein provides significant advantages over prior art dressing forms as it provides for wound healing in combination with haemostasis and contamination and infection control while avoiding antagonism between pro-healing, haemostatic and antimicrobial elements.

### Example I - Haemostatic and antimicrobial polyurethane foam preparations

To prepare haemostatic and antimicrobial foam the haemostat polyanhydroglucuronic acid (PAGA) (HemCon Medical Technologies Europe Ltd, Dublin) and the antimicrobial, compound chlorhexidine di-gluconate (CHG) (Napp Technologies LLC, New Jersey) were used. Polyurethane foam dressings were prepared with varying concentrations of PAGA and CHG relative to the final dry weight of polyurethane foam. The polyurethane foam used is type MS50P(w) Lendell medical foam available from Filtrona Porous Technologies (www.filtronaporoustechnologies.com)

| | |
|---|---|
| Usable Width: | 15 inches (381mm) |
| Thickness: | 0.22 inches (5.6mm) |
| % Moisture: | 2% |
| Density: | 6.0 pcf (96Kg/m³) |
| Tensile Strength : | 51.0 psi (352 kPa) |
| Target Elongation: | 194% |
| Tear Strength: | 5.6 pli (0.98kN/m) |
| CDF @ 50%: | 0.74 psi (5.14 kPa) |
| Durometer: | 47 shore |
| Cell Size: | 131 ppi |
| Absorption: | 15 g/g |
| Expansion: | 75% |
| Wrung Retention: | 1.2 g/g |

The polyurethane foam was produced by firstly producing a prepolymer comprising a polyisocyanate [OCN-R-NCO]ₙ and diol [OH-R-OH] which were mixed in a pre-polymer reaction vessel. The components of the pre-polymer were mixed together using agitation in a mechanical mixer for over ten minutes ensuring that all component were thoroughly mixed. The polyurethane polymerisation reaction occurred in the pre-polymer mixing vessel. In a separate vessel the PAGA and CHG were blended together in a vessel containing only water and surfactant with continual mixing until a homogenous suspension had been achieved. Unlike other haemostats the PAGA haemostat had the solubility and viscosity characteristics that allow for aqueous mixing and it additionally demonstrates chemical inertness towards the CHG and silver entities to allow such aqueous phase preparation. The water content of the aqueous phase ranged up to 300% stoichiometric equivalents to the pre-polymer. Surfactants chosen from the group silicone oils, polydimethylsiloxane-polyoxyalkylene block copolymers, nonylphenol ethoxylates, or other similar acting organic compounds used for the dual purpose of acting as anti foaming compounds in the aqueous phase while regulating the correct cell size and structure and overall physical appearance of the foam. The aqueous phase containing the actives and the reacted prepolymer mix were then both independently pumped to a third vessel where they were physically mixed by mechanical means ensuring a homogenous mixture. The pre-polymer and aqueous phase mixture was then dispensed from the mixing vessel onto a conveyer belt coated with a carrier liner to prevent adherence to the belt. The water of the aqueous phase reacted with the isocyanate groups of the pre-polymer and CO₂ gas was expelled which caused the foam to rise to desired height 0.375 inches. The polyurethane foam was then covered with a nitrogen blanket to prevent further reaction and allowed to cure and dry for 24 - 72 hrs. A number of different formulations were prepared for manufacturing suitability. The formulations with the impregnated components are outlined in Table 1.

**Table 1: Prepared foam formulations**

| **PAGA (w/w %)** | **CHG (w/w %)** | **Polyurethane (w/w %)** |
|---|---|---|
| 15 | 30 | 55 |
| 15 | 22.5 | 62.5 |
| 15 | 15 | 70 |
| 15 | 7.5 | 68.5 |
| 15 | 5 | 80 |
| 11.25 | 22.5 | 66.25 |
| 7.5 | 15 | 68.5 |
| 3.75 | 7.5 | 88.25 |

### Example 2 - Antibacterial Efficacy of prepared formulations calcium sodium salt polyanhydroglucuronic acid and Chlorhexidine di-gluconate in a polyurethane foam

Polyurethane foam matrix dressings were prepared with the calcium sodium salt of polyanhydroglucuronic acid (15% w/w) and w/w percentages of CHG at 0%, 5%, 11%, 15%, 23% and 30% as presented in Example 1. The dressing comprising w/w percentages of CHG at 0%, 5%, 23% and 30% are reference examples and are not encompassed by the present invention. All of the formulations were investigated for their antibacterial efficacy against methicillin-resistant Staphylococcus aureus (MRSA) using AATCC Test Method 100 "Assessment of Antibacterial Finishes on Textiles".

Analysis of Figure 1 indicates that the acceptable minimum low range of chlorhexidine di-gluconate percentage weight fraction in the polyurethane foam matrix is 9% (20 mg) to 16% (35 mg) w/w since this range achieves the acceptable >Log 4 reduction.

The results for gamma-irradiated sterilized testing and non gamma-irradiated testing are presented in Table 2.

**Table 2: Formulations of PAGA impregnated PU foam with increasing CHG concentrations**

| **CHG** | **5% (w/w)** | **11% (w/w)** | **15% (w/w)** | **23% (w/w)** | **30% (w/w)** |
|---|---|---|---|---|---|
| **Log Reduction (Sterile)** | 2.3 | >4.7 | 5.3 | >5.4 | >5.0 |
| **Log Reduction (Non-sterile)** | 2.7 | >5.2 | >5.3 | >5.4 | 5.3 |

### Example 3 - Device assembly

A catheter access site dressing device to control bleeding was prepared by impregnating calcium sodium salt of polyanhydroglucuronic acid into polyurethane foam. CHG was incorporated to achieve an antimicrobial efficacy of greater than 4 log in 24 hours. A formulation as described in Table 3 was prepared and a moisture vapour permeable backing that comprised of polyurethane film with a MVTR of 1000 gm/m²/24hr (3M) was adhered.

**Table 3; IV site device composition**

| **Ingredients** | **Formulation** (% w/w) final formulation |
|---|---|
| Chlorhexidine gluconate | 11 |
| Calcium-sodium polyanhydroglucuronic acid | 8 |
| Hydrophillic flexible polyurethane foam | 81 |

The polyurethane foam matrix was die cut into 25. mm diameter disks with a central 4 mm diameter section removed from each disk. A radial slit was also punched from the centre of the disk to the outside of the disk. The slit and 4 mm punch are designed to allow catheter access. The dressing is sterilized by gamma irradiation between 25 and 45 kGy, sufficient to produce a sterility assurance limit (SAL) of 10⁻⁶.

The device described was tested for antimicrobial efficacy against a number of micro-organisms including gram positive and gram negative bacteria, fungi and yeast. The antimicrobial efficacy was tested using the AATCC Test Method 100 "Assessment of Antibacterial Finishes on Textiles". In summary 1.0 ml of test organism suspension at a minimum of 1 x 10⁶ CFU / ml was inoculated to the test sample. At selected time points (time zero and 24 hours) organisms were extracted in a neutralizer media (D/E broth) which was diluted and plated. Log reduction and percent reduction were determined. The results obtained are shown in Table 4.

**Table 4: Antimicrobial results of the IV site device**

| **Micro-organism** | **Decrease of CFU number / 24 hours** |
|---|---|
| Staphylococcus aureus | > 4 log |
| Staphylococcus epidermidis | > 4 log |
| Enterococcus faecium | > 4 log |
| Escherichia coli | > 4 log |
| Pseudomonas aeruginosa | > 4 log |
| Acinetobacter baumanii | > 4 log |
| Klebsiella pneumoniae | > 4 log |
| Candida albicans | > 4 log |
| Aspergillus niger | > 4 log |

### Example 4 - In vitro Haemostatic Efficacy

The device described in Example 3 was tested for its ability to activate the intrinsic blood coagulation cascade, specifically coagulation factor XIIa and kallikrein. In summary, 0.5 cm² of the device and also a control device which was another polyurethane IV site device but without polyanhydroglucuronic acid (1" DISK,4.0 mm centre hole with radial slit and containing 92 mg CHG (Biopatch; Ethicon)) ,were placed in Eppendorfs. 0.25 ml of deionised H₂O was added to the dressings and incubated at room temperature for 10 min. After 10 min incubation, the dressings were compressed and the fluid supernatant removed. Subsequently, 45 ul of the fluid supernatant was added to fresh Eppendorfs. Then 90 ul of deionised H₂O was added along with 45 ul of normal coagulation control plasma. The samples were mixed and incubated at room temperature for 10 min. After the incubation stage 40 ul of each sample were added to microtitre plate wells and 40 ul of 0.8 mM S-2302 (specific Factor XIIa and kallikrein chromogenic substrate) was then added to initiate the reaction. The reaction was allowed to proceed for 3 minutes and then the optical density at 405 nm was read. The results for this study are presented in Table 5.

**Table 5 Activation of Factor XIIa and kallikrein**

| **Sample** | **Mean Optical Density @ 405 nm (3 min read)** | **Activity Rate/min** |
|---|---|---|
| PAGA containing PU Foam IV device | 53.40 | 17.80 |
| Other non PAGA containing PU Foam IV device | 0 | 0 |

The control IV site dressing not containing PAGA did not activate the coagulation factor XIIa and kallikrein of the intrinsic coagulation system. The device described by Example 3 did activate the intrinsic coagulation enzymes. Such activation is consistent with oxidized cellulose mechanism of action and this demonstrated the potential of the device to be a haemostat.

### Example 5 - Haemostatic Efficacy - In vivo measurements

Having established the potential for haemostatic activity in Example 4 the device was tested for haemostatic activity in a suitable in vivo bleeding model. Devices of the formulation as described in Example 3 were tested for their haemostatic efficacy *in vivo* in a rabbit ear model. The study was divided in two periods. Within the first test period (D +1) the Test Item was tested on the left ear of the rabbit, the right ear was used as control. Within the second period (D +3) the Test Item was tested on the right ear of the rabbit, the left ear was used as a control. Bleeding was caused by puncture of a lateral ear vein with an infection needle (external diameter always 0.9 mm). On D +1 the puncture was performed at an acral part of the ear, on D +3 the puncture was performed cranially. Distance between both punctures was 2 - 3 cm. The test and control were applied immediately after the puncture wounds were made. Test items and controls were weighed before their use and immediately after cessation of bleeding. Also the time from start to the end of bleeding was measured.

In this study wounds treated with the Test Item bled for a shorter period of time and had a smaller blood loss compared to the control (Pur-Zellin® cellulose swab, HARTMANN-RICO a.s.) thereby demonstrating the haemostatic efficacy of the device. Data demonstrating the in vivo haemostatic efficacy of the device is outlined in Table 7.

**Table 11 Results for the device in time to stop bleeding and blood loss mass**

| | **Test Item (n=16)** | **Control (n=16)** |
|---|---|---|
| **Average quantity of Absorbed Blood (g)** | 0.167 ± 0.18 | 1.311 ± 1.08 |
| **Average Time of Bleeding (seconds)** | 48.8 ± 20.1 | 89.4 ± 77.4 |

### Example 6 - Wound Healing

The effect on wound healing of the device prepared with the composition of Example 3 was assessed on dermal wound healing in two separate in vivo studies on rats.

Dressings prepared with the composition of Example 3 were assessed in vivo for their affect on dermal wound healing in rats. Each of twelve animals received three dorsal full thickness wounds created with an 8mm dermal punch. Following wound creation the wound was covered with a test sample, a control dressing (non PAGA containing IV site dressing as in Example 4) or left untreated. The wound sites on each animal were covered with a secondary dressing. Animals were observed daily to ensure integrity of the wound, to observe signs of general clinical health and to record wound measurements. The same dressing that was removed was replaced on the wound after each measurement had been taken. Dressings were changed as necessary depending on the degree of saturation with exudate and wear time was limited to a maximum of 7 days exposure of a single treatment on the wound.

All wounds healed comparably by day 14 with the test article of the composition of Example 3 performing between the untreated wound (see Figure 5) and the control dressing. However, it could be seen that during the midstage of the study the animals from the control dressing group showed slower dermal healing compared to the described device and the negative control. This can be attributed to the significantly higher CHG content (92 mg/dressing or 30% (w/w)) of the control dressing.

### Example 7 - Further Wound Healing and Oedema formation

Dressings prepared with the composition of Example 3 were assessed in vivo for their affect on dermal wound healing in rats in an experiment similar to that described in Example 6. Each of ten animals received three dorsal full thickness wounds to the depth of the subcutis created with a 10mm dermal punch. Following wound creation each of the three wound on each animal was covered with either a test sample, a control dressing (non PAGA containing IV site dressing as in Example 4 and 6) or left untreated. The wound sites on each animal were covered with a secondary dressing. Animals were observed daily to ensure integrity of the wound, to observe signs of general clinical health and to record wound measurements. The same dressing that was removed was replaced on the wound after each measurement had been taken. Dressings were changed as necessary depending on the degree of saturation with exudate and wear time was limited to a maximum of 7 days exposure of a single treatment on the wound. The wounds were also evaluated for signs of erythema and oedema.

As with the study described in Figure 9 all wounds healed comparably by day 10 with the test article of the composition of Example 3 performing between the untreated wound and control dressing (See Figure 6). There were no visible signs of erythema development at any of the wound sites (Table 12). Slight oedema formation was reported for untreated wounds and those treated with the test item (Figure 7 and Table 13). In general a similar response was observed for un-treated wounds and wounds treated with the test item. Oedema formation was more pronounced in wounds treated with the control dressing which contained a significantly higher fraction of CHG (30 %w/w).

Generally, untreated wounds and wounds treated with the test item healed in similar manners. Both healed at a faster rate than wounds treated with control dressing and the higher CHG concentrations. Also Oedema formation was less pronounced in these wounds compared to wounds treated with control dressing. The less favorable wound healing results seen for the control dressing can be attributed to the higher CHG content (30 %w/w).

**Table 12: Erythema Formation**

| **Erythema Average Score** | | | | | | | | | | | |
|---|---|---|---|---|---|---|---|---|---|---|---|
| **Day** | **0** | **1** | **2** | **3** | **4** | **5** | **6** | **7** | **8** | **9** | **10** |
| **Un-treated** | 1 | 0 | 0 | 0 | 0 | 0 | 0 | 0 | 0 | 0 | 0 |
| **Test Item** | 1 | 0 | 0 | 0 | 0 | 0 | 0 | 0 | 0 | 0 | 0 |
| **Control Dressing** | 1 | 0 | 0 | 0 | 0 | 0 | 0 | 0 | 0 | 0 | 0 |

| | | | | | | | | | | | |
|---|---|---|---|---|---|---|---|---|---|---|---|
| Key: (0 = Normal (no erythema), 1 = Slight erythema, 2 = Mild erythema, 3 = Severe erythema) | | | | | | | | | | | |

**Table 13: Oedema Development**

| **Oedema Average Score** | | | | | | | | | | | |
|---|---|---|---|---|---|---|---|---|---|---|---|
| **Day** | **0** | **1** | **2** | **3** | **4** | **5** | **6** | **7** | **8** | **9** | **10** |
| **Un-treated** | 1 | 1 | 1.3 | 1.2 | 1.1 | 1.1 | 1.1 | 1.1 | 1.1 | 1.1 | 1 |
| **Test Item** | 1 | 1 | 1.1 | 1.7 | 1.3 | 1.3 | 1.3 | 1.1 | 1.1 | 1.1 | 1.1 |
| **Control Dressing** | 1.1 | 1 | 1.3 | 2 | 1.8 | 2 | 2 | 1.8 | 1.8 | 1.8 | 1.9 |
| Key: (0 = Normal (No oedema), 1 = Slight oedema, 2 = Mild oedema, 3 = Severe oedema) | | | | | | | | | | | |

### Example 8 - Sustained Antimicrobial Efficacy - Log Reduction

To demonstrate the sustained antimicrobial efficacy of was dressing formulation in example 3 over 24 hours and 7 days, AATCC Test Method 100-2004 "Assessment of Antimicrobial Finishes on Textiles" was used. The results of this testing (Table 14) demonstrate that the formulation in example 3 is highly effective in controlling a broad range of gram negative and gram positive bacteria as well as the fungi C. albicans and A. niger. Also a modified version of the AATCC Test Method 100 was investigated. In the modified AATCC 100 test method, in addition to testing dressing samples following 24 hour exposure to the test organisms, reference and test dressings are also exposed for 6 days to a mock wound environment that potentially could lead to loss or degradation of the antimicrobial activity. Following the 6-day exposure, the dressings are inoculated and the test conducted according to the standard AATCC Test Method 100. Dressing were tested against a number of micro-organisms including gram positive and gram negative bacteria and dimorphic fungi / yeast. The log reduction data observed following 24 hours and 7 days is outlined in Table 15 below. A log reduction of greater than 4 log was recorded for each of the test organisms demonstrating the sustained antimicrobial activity of the antimicrobial agent in the dressing.

**Table 14 Standard AATCC antimicrobial finish testing**

| **Micro-organism** | **24 hrs Log Reduction** | **7 days Log Reduction** |
|---|---|---|
| Staphylococcus aureus CCM 7110 | 5.50 | 6.31 |
| Staphylococcus epidermidis CCM 7221 | 5.53 | 6.18 |
| Enterococcus faecium CNCTC 5773 | 5.52 | 5.51 |
| Escherichia coli CCM 4517 | 5.58 | 6.38 |
| Pseudomonas aeruginosa CCM 1961 | 5.76 | 6.70 |
| Acinetobacter baumanii CNCTC 6168 | 5.55 | 6.16 |
| Klebsiella pneumoniae CCM 4415 | 4.83 | 6.62 |
| Candida albicans CCM 8215 | 4.72 | 4.71 |
| Aspergillus niger | 4.20 | 4.19 |

**Table 15 Modified AATCC antimicrobial finish testing**

| **Micro-organism** | **7 days Log Reduction** |
|---|---|
| Staphylococcus aureus CCM 7110 | >4 |
| Staphylococcus epidermidis CCM 7221 | >4 |
| Enterococcus faecium CNCTC 5773 | >4 |
| Escherichia coli CCM 4517 | >4 |
| Pseudomonas aeruginosa CCM 1961 | >4 |
| Acinetobacter baumanii CNCTC 6168 | >4 |
| Klebsiella pneumoniae CCM 4415 | >4 |
| Candida albicans CCM 8215 | >4 |
| Aspergillus niger | >4 |

### Example 9 - Sustained Antimicrobial Efficacy -Zone of Inhibition

A Kirby-Bauer Zone of Inhibition method was used to investigate the sustained antimicrobial efficacy of the dressing in Example 3 over 24 hours and 7 days. Overnight cultures were prepared to a minimum inoculum count of 1 x 10⁷ CFU / ml and spread on freshly prepared agar plates. An individual test article was placed onto the agar plate and incubated for 24 hrs at 35-37°C. The area under the test article was swabbed and the swab was transferred onto sterile agar plates. The test article was then placed on a freshly inoculated agar plate and the procedure repeated. The test articles were transferred each day for up to seven days. Growth from the swabs taken from the test articles indicated Bacteriostatic action of the antimicrobial agent while no growth indicated bacteriocidal action. Samples were tested in triplicate. The bacteriocidal or bacteriostatic action of the dressing at 7 days is shown in Table 16. Figure 2 shows zone of inhibition results at 24 hrs while Figures 3A & 3B show the zone of inhibition %changes at 1, 2, 3, 4, 5, 6 & 7 days.

**Table 16 Bacteriocidal or Bacteriostatic action of the device**

| **Micro-organism** | **Bacteriocidal or Bacteriostatic** |
|---|---|
| Staphylococcus aureus CCM 7110 | Bacteriocidal |
| Staphylococcus epidermidis CCM 7221 | Bacteriocidal |
| Enterococcus faecium CNCTC 5773 | Bacteriocidal |
| Escherichia coli CCM 4517 | Bacteriocidal |
| Pseudomonas aeruginosa CCM 1961 | Bacteriostatic |
| Acinetobacter baumanii CNCTC 6168 | Bacteriostatic |
| Klebsiella pneumoniae CCM 4415 | Bacteriocidal |
| Candida albicans CCM 8215 | Bacteriostatic |

### Example 10 - A Prospective Human Clinical Study of Suppression of Skin Microflora

The primary objective of this study was to investigate the ability of the polyurethane foam matrix dressing formulation of example 3 to suppress the regrowth of skin microflora following skin preparations on healthy human volunteers. This study was performed on healthy human volunteers following the method of Maki et al. 2008. The study was independently conducted by the Center for Laboratory Activities in Public Health Protection and Promotion, National Reference Laboratory for Disinfection and Sterilization, National Institute of Health, Prague, Czech Republic.

Subjects - A group of 12 study subjects was selected and enrolled for testing through informed consent. All were Caucasian with an average age of 52.5 years and an age range between 25 years and 69 years. This study was conducted to assess the capacity of the test dressings (example 3 formulation) to suppress skin flora re-growth following skin prepping for 1 minute with 70% isopropyl alcohol when compared to an inactive control dressing. Each subject served as his or her own control by using 8 randomized sites in the subclavian area of each volunteer. On study day 0, baseline skin flora counts were established from randomized sites. Skin flora count from these randomized sites was also measured following air drying immediately post-prep with 70% isopropyl alcohol. Once the remaining sites had air-dried immediately post-prep, the test dressings (example 3 formulation) and the control dressings (polyurethane foam with no CHG or oxidized cellulose) were applied to the remaining prepped sites of the subjects. Dressings were applied to the subclavian sites using sterile tweezers and attached by latex-free, hypoallergenic and transparent polyurethane securement dressings. The dressings were left up to 10 days, and skin flora counts were taken at 7 and 10 day time points. Skin flora was measured using standard scrubbing techniques and the skin flora beneath the dressing quantitated through use of a recovery solution that was then cultured on agar plates. Wilcoxon paired tests were used for statistical testing of the level of significance (P-values <0.05 were considered significant).

Disinfection of the skin prior to catheter insertion provides substantial protection to a site, but viable bacteria may still remain on the skin and re-grow over time, thus leading to a greater possibility of infection. Any catheter related bloodstream infection preventive strategy should be able to reduce skin microbial colonization for the duration of the catheter insertion. The results seen in Figure 4 show the effect of the 70% isopropyl alcohol skin prep. The raw skin flora counts were dramatically reduced, as would be expected. It can also be observed that after both the 7 day and 10 day time points, the test dressings maintained the skin flora at levels equivalent to those of the post-prep level, whereas with the control dressings significant skin flora re-growth was evident. Bacterial counts were converted to log10 CFU /cm2 prior to statistical analysis. At day 7, the test dressings showed significantly lower skin flora counts post-prep compared to the control dressings which had substantial re-growth (P<0.001). At day 10, test dressings also showed significantly lower re-growth (P<0.001). As can be seen (Figure 4), the test dressing maintained the skin flora count at less than the post-prep count for the complete duration of the study out to 10 days.

No adverse events, such as skin irritation, edema or erythema formation were reported for the study with the test dressing. The test dressing successfully and significantly prevented the re-growth of microorganisms for up to 10 days as demonstrated by this study. After both 7 and 10 days, the microbial count was seen to be less than that of the post-prep microbial count. As such, it would be expected that the test dressing formulation (example 3) would be an effective component of a strategy to reduce skin microbial colonization. From literature, such a reduction in skin colonization markedly reduces the risk of catheter related bloodstream infection [Bjornson et al. 1982, Safdar et al. 2004, Maki et al. 1997].

### Example 11- Different Physical Embodiments

The produced PAGA and CHG impregnated foam described in Example 3 was also die cut into different sized and shaped devices. Radial slits were always punched from the centre of the disk to the outside of the device but different catheter access site holes and shapes were produced. Some of these different physical embodiments of the device can be seen in Figure 8.

In Fig. 8(a) the device has a diameter of 1 inch (2.54cm) with a 1.5mm central access site hole and a radial slit extending outwardly from the central hole.

The device of Fig. 8(b) is similar to 8(a) but in this case there is a 4mm central hole.

The device of Fig. 8(c) is also similar to 8(a) but in this case there is a 7mm central hole.

The device of Fig. 8(d) is similar to 8(a) but in this case there is a T-shaped central access site.

The device of Fig. 8(e) has a + shaped access site whilst the device of Fig. 8(f) has an X shaped access site.

The device of Fig. 8(g) is an orthogonal shaped device with a central access site hole which may be about 4mm and there is a radial slit.

Fig. 8(b) shows a rectangular shaped device with a central access site hole which may be about 4mm and again in this case there is a radial slit.

### Example 12 - Microscopy analysis of foam constructs

The PAGA and CHG impregnated foam dressings were also studied using microscopy to so demonstrate the impregnation of the dressing with PAGA. Thin sections of the dressing were cut with a scalpel and placed into wells of 6-well plates. 1 ml aliquots of a solution of 0.001 % aqueous bromophenol blue were added to the well and allowed to stain at room temperature (RT) for 30 min. As a Negative Control, a thin section of non impregnated foam dressing which did not contain PAGA, were similarly treated, Bromophenol blue is an acid phthalein dye, commonly used as a pH indicator and was used here for better visualisation contrast of the polyurethane and PAGA due to their different pHs.

After staining for 30 min, the bromophenol blue was removed and the sections of the dressings were washed with 3 ml deionised H₂O. The washing was repeated three times. Images of the dressings were taken using an Olympus CKX41 microscope with an Olympus E-600 digital camera attached at a magnification of 10x. Representative images are presented in Figure 9. Figure 9A) shows the standard foam without active impregnation. The stained micrograph shows the cell structure of the individual cell units. Figure 9B) shows the PAGA impregnated foam. The stained PAGA particles can be seen in the micrograph along with the polyurethane foam stained cells.

Although the disclosure hereof is detailed and exact to enable those skilled in the art to practice the invention, the physical embodiments herein disclosed merely exemplify the invention that may be embodied in other ways. While the preferred embodiment has been described the details may be changed without departing from the invention.

Modifications and additions can be made to the embodiments of the invention described herein without departing from the scope of the invention. For example, while the embodiments described herein refer to particular features, the invention includes embodiments having different combinations of features. The invention also includes embodiments that do not include all of the specific features described.

### References

Mital A, Fried LF and Piraino B, 2004 "Bleeding complications associated with peritoneal dialysis catheter insertion" Peritoneal Dialysis Int. 24 p 478 - 480
Doerfler ME, Kaufman B and Goldenberg AS, 1996 "Central venous catheter placement in patients with disorders of hemostasis" Chest 110 (1) p 185 - 188
Bouza E, Burillo A and Munoz, 2002 "Catheter-related infections: diagnosis and intravascular treatment" Clin. Microbiol. Infect. 8: 265 - 274
Maki DG, Mermel LA 1998 "Infections due to infusion therapy" Taken from: Bennett JV, Brachman PS, eds. Hospital Infections. Philadelphia, Pa: Lippincott-Raven; p 689-724
Raad II, Hanna HA and Darouiche RO 2001 "Diagnosis of Catheter -Related Bloodstream Infections: Is it necessary to culture the subcutaneous catheter segment?" Eur. J. Clin. Microbiol. Infect. Dis. 20 p 566 - 568
Sherertz RJ, Heard SO and Raad II, 1997 "Diagnosis of triple-lumen catheter infection: Comparison of roll plate, sonication and flushing methodologies" J. Clin. Microbiol. 35 p 641-646
Timsit JF, 2007 "Diagnosis and prevention of catheter-related infections" Curr. Opin. Crit. Care 13 (5) p 563 - 571
Lee R1 and White PD "A Clinical Study of the Coagulation Time of Blood" J. Am. Med. Sci., Apr. 1913 Vol. 245 (4), 495 - 503.
Maki et al. "Novel Integrated Chlorhexidine-impregnated Transparent Dressing for Prevention of Vascular Catheter-related Bloodstream Infection: A Prospective Comparative Study in Healthy Volunteers", Poster Presentation at the Society for HealthCare Epidemiology of America Conference 2008, Orlando, F1.
Bjornson, H.S., et al. 1982 "Association between microorganism growth at the catheter insertion site and colonization of the catheter in patients receiving total parenteral nutrition" Surgery, 92(4): p. 720-7.
Safdar, N. and D.G. Maki 2004 "The pathogenesis of catheter-related bloodstream infection with noncuffed short-term central venous catheters" Intensive Care Med, 30(1): p. 62-7.
Maki, D.G., et al. 1997 "Prevention of central venous catheter-related bloodstream infection by use of an antiseptic-impregnated catheter. A randomized, controlled trial" Ann Intern Med, 127(4): p. 257-66

## Claims

1. A wound dressing device for use with a transcutaneous medical device such as a cannula or a catheter, the dressing device comprising a flexible hydrophillic polyurethane matrix, an antimicrobial agent contained within the matrix, and a haemostatic agent contained within the matrix, the haemostatic agent comprising polyanhydroglucuronic acid or salt thereof in an amount to achieve a haemostatic effect, and the antimicrobial agent comprising chlorhexidine di-gluconate in an amount to achieve an antimicrobial effect without adversely affecting wound healing, wherein the chlorhexidine di- gluconate is present in an amount of from 9% to 16% (w/w).

2. A wound dressing device as claimed in claim 1 wherein the polyanhydroglucuronic salt is present in an amount of from 3% to 20% (w/w).

3. A wound dressing device as claimed in claim 1 or claim 2 wherein the polyanhydroglucuronic salt is present in an amount of approximately 8% (w/w).

4. A wound dressing device as claimed in any one of claims 1 to 3 wherein the chlorhexidine di- gluconate is present in an amount of approximately 11 % (w/w).

5. A wound dressing device as claimed any of claims 1 to 4 comprising approximately 8% (w/w) polyanhydroglucuronic acid, approximately 11 % (w/w) chlorhexidine di-gluconate, and approximately 81% hydrophillic flexible polyurethane foam.

6. A wound dressing device as claimed in any of claims 1 to 5 comprising an aperture for reception of a medical device such as a cannula or a catheter.

7. A wound dressing device as claimed in any one of claims 1 to 6 comprising a breathable backing material to allow vapour transmission from the device.

8. A wound dressing as claimed in any one of claims 1 to 7 wherein a skin contacting side of the device contains an adhesive to retain the device affixed to a site.

9. A wound dressing device as claimed in any one of claims 6 to 8 wherein the central access aperture is a circular hole ranging in size from 0.1 mm to 10mm in diameter.

10. A wound dressing device as claimed in any one of claims 6 to 8 wherein the central access aperture is "x" shaped.

11. A wound dressing device as claimed in any one of claims 6 to 8 wherein the central access aperture is "T" shaped.

12. A wound dressing device as claimed in any one of claims 1 to 11 wherein the device contains a quantity of the antimicrobial agent to maintain antimicrobial efficiency for up to 7 days.

13. A wound dressing device as claimed in any one of claims 1 to 12 in conjunction with a transcutaneous medical device such as a cannula or a catheter.

## Patentansprüche

1. Wundauflagevorrichtung zur Verwendung mit einer transkutanen medizinischen Vorrichtung wie etwa einer Kanüle oder einem Katheter, wobei die Auflagevorrichtung eine flexible hydrophile Polyurethanmatrix, ein antimicrobielles Mittel, das in der Matrix enthalten ist, und ein hämostatisches Mittel, das in der Matrix enthalten ist, umfasst, wobei das hämostatische Mittel Polyanhydroglucuronsäure oder ein Salz davon in einer Menge umfasst, die einen hämostatischen Effekt erzielt, und das antimicrobielle Mittel Chlorhexidin-di-gluconat in einer Menge umfasst, die einen antimicrobiellen Effekt erzielt, ohne die Wundheilung nachteilig zu beeinflussen, worin das Chlorhexidin-di-gluconat in einer Menge von 9 % bis 16 % (Gewicht/Gewicht) vorliegt.

2. Wundauflagevorrichtung gemäß Anspruch 1, worin das Polyanhydroglucuronsäuresalz in einer Menge von 3 % bis 20 % (Gewicht/Gewicht) vorliegt.

3. Wundauflagevorrichtung gemäß Anspruch 1 oder Anspruch 2, worin das Polyanhydroglucuronsäuresalz in einer Menge von ungefähr 8 % (Gewicht/Gewicht) vorliegt.

4. Wundauflagevorrichtung gemäß einem der Ansprüche 1 bis 3, worin das Chlorhexidin-di-gluconat in einer Menge von ungefähr 11 % (Gewicht/Gewicht) vorliegt.

5. Wundauflagevorrichtung gemäß einem der Ansprüche 1 bis 4, umfassend ungefähr 8 % (Gewicht/Gewicht) Polyanhydroglucuronsäure, ungefähr 11 % (Gewicht/Gewicht) Chlorhexidin-di-gluconat und ungefähr 81 % hydrophilen flexiblen Polyurethanschaum.

6. Wundauflagevorrichtung gemäß einem der Ansprüche 1 bis 5 umfassend eine Öffnung zur Aufnahme einer medizinischen Vorrichtung wie etwa einer Kanüle oder eines Katheters.

7. Wundauflagevorrichtung gemäß einem der Ansprüche 1 bis 6 umfassend ein atmungsaktives Trägermaterial, um Dampfdurchlässigkeit aus der Vorrichtung zu erlauben.

8. Wundauflage gemäß einem der Ansprüche 1 bis 7, worin eine Hautkontaktseite der Vorrichtung einen Klebstoff enthält, um die Vorrichtung an einer Stelle befestigt zu halten.

9. Wundauflagevorrichtung gemäß einem der Ansprüche 6 bis 8, worin die zentrale Zugangsöffnung ein kreisförmiges Loch ist, dessen Größe im Bereich von 0,1 mm bis 10 mm im Durchmesser liegt.

10. Wundauflagevorrichtung gemäß einem der Ansprüche 6 bis 8, worin die zentrale Zugangsöffnung x-förmig ist.

11. Wundauflagevorrichtung gemäß einem der Ansprüche 6 bis 8, worin die zentrale Zugangsöffnung T-förmig ist.

12. Wundauflagevorrichtung gemäß einem der Ansprüche 1 bis 11, worin die Vorrichtung eine Menge des antimikrobiellen Mittels enthält, um die antimikrobielle Wirksamkeit für bis zu 7 Tage aufrechtzuerhalten.

13. Wundauflagevorrichtung gemäß einem der Ansprüche 1 bis 12 in Verbindung mit einer transkutanen medizinischen Vorrichtung wie etwa einer Kanüle oder einem Katheter.

## Revendications

1. Dispositif de pansement pour plaie pour une utilisation avec un dispositif médical transcutané tel qu'une canule ou un cathéter, le dispositif de pansement comprenant une matrice de polyuréthane hydrophile souple, un agent antimicrobien contenu dans la matrice, et un agent hémostatique contenu dans la matrice, l'agent hémostatique comprenant de l'acide polyanhydroglucuronique ou un sel de celui-ci en une quantité permettant obtenir un effet hémostatique, et l'agent antimicrobien comprenant du digluconate de chlorhexidine en une quantité permettant d'obtenir un effet antimicrobien sans affecter négativement la cicatrisation de plaie, dans lequel le digluconate de chlorhexidine est présent en une quantité allant de 9% à 16% (w/w).

2. Dispositif de pansement pour plaie tel que revendiqué dans la revendication 1, dans lequel le sel d'acide polyanhydroglucuronique est présent en une quantité allant de 3% à 20% (w/w).

3. Dispositif de pansement pour plaie tel que revendiqué dans la revendication 1 ou 2, dans lequel le sel d'acide polyanhydroglucuronique est présent en une quantité d'environ 8% (w/w).

4. Dispositif de pansement pour plaie tel que revendiqué dans l'une quelconque des revendications 1 à 3, dans lequel le digluconate de chlorhexidine est présent en une quantité d'environ 11% (w/w).

5. Dispositif de pansement pour plaie tel que revendiqué dans l'une des revendications 1 à 4, comprenant environ 8% (w/w) d'acide polyanhydroglucuronique, environ 11% (w/w) de digluconate de chlorhexidine, et environ 81% de mousse de polyuréthane souple hydrophile.

6. Dispositif de pansement pour plaie tel que revendiqué dans l'une des revendications 1 à 5, comprenant une ouverture pour la réception d'un dispositif médical tel qu'une canule ou un cathéter.

7. Dispositif de pansement pour plaie tel que revendiqué dans l'une quelconque des revendications 1 à 6, comprenant un matériau d'appui perméable à l'air pour permettre la transmission de la vapeur à partir du dispositif.

8. Dispositif de pansement pour plaie tel que revendiqué dans l'une quelconque des revendications 1 à 7, dans lequel un côté en contact avec la peau du dispositif contient un adhésif pour garder le dispositif apposé sur un site.

9. Dispositif de pansement pour plaie tel que revendiqué dans l'une quelconque des revendications 6 à 8, dans lequel l'ouverture d'accès centrale est un trou circulaire dont la taille du diamètre varie de 0,1 mm à 10 mm.

10. Dispositif de pansement pour plaie tel que revendiqué dans l'une quelconque des revendications 6 à 8, dans lequel l'ouverture d'accès centrale est en forme de "x".

11. Dispositif de pansement pour plaie tel que revendiqué dans l'une quelconque des revendications 6 à 8, dans lequel l'ouverture d'accès centrale est en forme de "T".

12. Dispositif de pansement pour plaie tel que revendiqué dans l'une quelconque des revendications 1 à 11, dans lequel le dispositif contient une quantité de l'agent antimicrobien permettant de maintenir une efficacité antimicrobienne jusqu'à 7 jours.

13. Dispositif de pansement pour plaie tel que revendiqué dans l'une quelconque des revendications 1 à 12 conjointement avec un dispositif médical transcutané tel qu'une canule ou un cathéter.
